(19) Europäisches Patentamt
European Patent Office
Office européen des brevets

(11) **EP 2 949 320 A1**

(12) **EUROPÄISCHE PATENTANMELDUNG**

(43) Veröffentlichungstag:
**02.12.2015 Patentblatt 2015/49**

(51) Int Cl.:
***A61K 9/16*** *(2006.01)* ***A61K 47/12*** *(2006.01)*

(21) Anmeldenummer: **15172814.4**

(22) Anmeldetag: **12.01.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **17.01.2008   DE 102008004893**

(62) Dokumentnummer(n) der früheren Anmeldung(en) nach Art. 76 EPÜ:
**09702374.1 / 2 244 696**

(71) Anmelder: **ADD Advanced Drug Delivery Technologies, Ltd.**
**4133 Pratteln (CH)**

(72) Erfinder:
• **WEIGT, Antje**
**01277 Dresden (DE)**

• **KEMPE, Wolfgang**
**01169 Dresden (DE)**
• **SCHLÜTERMANN, Burkhard**
**79280 Au (DE)**

(74) Vertreter: **Pfenning, Meinig & Partner GbR**
**Patent- und Rechtsanwälte**
**Theresienhöhe 11a**
**80339 München (DE)**

Bemerkungen:
Diese Anmeldung ist am 19-06-2015 als Teilanmeldung zu der unter INID-Code 62 erwähnten Anmeldung eingereicht worden.

(54) **TRÄGERPELLETS, VERFAHREN ZU DEREN HERSTELLUNG UND DEREN VERWENDUNG**

(57)    Die Erfindung betrifft ein Verfahren zur Herstellung von Trägerpellets für arzneiliche Wirkstoffe. Ebenso betrifft die Erfindung derartige Trägerpellets, sowie diese enthaltende pharmazeutische Formulierungen. Verwendung finden die erfindungsgemäßen Trägerpellets für den Transport und die Freisetzung von arzneilichen Wirkstoffen, insbesondere im menschlichen Körper.

EP 2 949 320 A1

**Beschreibung**

Die Erfindung betrifft ein Verfahren zur Herstellung von Trägerpellets für arzneiliche Wirkstoffe. Ebenso betrifft die Erfindung derartige Trägerpellets, sowie diese enthaltende pharmazeutische Formulierungen. Verwendung finden die erfindungsgemäßen Trägerpellets für den Transport und die Freisetzung von arzneilichen Wirkstoffen, insbesondere im menschlichen Körper.

Pharmazeutische, insbesondere per os anwendbare Darreichungsformen sollen für die jeweilige Anwendung in geeigneter Weise formuliert sein, um eine Freisetzung der pharmazeutischen Wirkstoffe zum richtigen Zeitpunkt und ohne störende Nebeneffekte zu bewirken. So sollen beispielsweise oral verabreichbare Wirkstoffe möglichst so freigesetzt werden, dass ein unangenehmer, z.B. bitterer Geschmack im Mund vermieden wird, da dieser insbesondere bei Kindern zu Abwehrreaktionen führen kann. Andererseits müssen die Wirkstoffe im Magen oder Darm möglichst vollständig und in raschresorbierbarer Form freigesetzt werden, wenn eine systemische Behandlung angestrebt wird.

Bei der oralen Verabreichung von Arzneimitteln wird der Wirkstoff im Magen-Darm-Trakt freigesetzt und ein Teil des Wirkstoffs resorbiert. Durch Steuerung der Freisetzung des Wirkstoffs kann das Ausmaß der Resorption und die Wirkungsdauer beeinflusst werden. Entsprechend sind verschiedene Vorschläge gemacht worden, um die Wirkstofffreisetzung durch geeignete galenische Formulierungen des Wirkstoffs zu steuern.

Ein Ansatz besteht darin, Darreichungsformen mit Überzügen zu versehen, wobei die Wirkstofffreisetzung abhängig von der Löslichkeit oder Durchlässigkeit der Überzüge beeinflusst werden kann. Derartige Überzüge können beispielsweise auf Tabletten oder Kapseln aufgebracht werden. In diesem Fall besteht jedoch ein Nachteil darin, dass ein fehlerhafter oder beschädigter Überzug dazu führen kann, dass die Freisetzung der gesamten Wirkstoffdosis nicht in der gewünschten Weise gesteuert wird.

Als Alternative bieten sich multipartikuläre Darreichungsformen an, bei denen die Gesamtmenge des Wirkstoffs auf eine größere Anzahl kleinerer Einheiten, wie Pellets, verteilt ist. Werden die einzelnen Pellets mit Überzügen versehen, so unterliegt im Fall eines fehlerhaften Überzugs bei einem Pellet nur ein entsprechend geringer Anteil der gesamten Wirkstoffdosis nicht der gewünschten Freisetzung.

Ein weiterer Vorteil derartiger Darreichungsformen auf der Basis von Pellets besteht darin, dass hinreichend kleine Pellets nach Einnahme relativ rasch aus dem Magen in den Darm übertreten. Tabletten hingegen können, sofern sie nicht zerfallen, auch für längere Zeit im Magen verbleiben, wobei die Zeit zudem recht variabel ist.

Bekannte Darreichungsformen mit kontrollierter Freisetzung sind somit nicht völlig zufrieden stellend. Zudem besteht das Problem, dass sich gewünschte (vorgegebene) Freisetzungsprofile in der Regel nicht einstellen lassen. Ferner ist die Herstellung von Darreichungsformen mit kontrollierter Freisetzung oftmals schwierig. Es besteht somit ein Bedarf an neuen Darreichungsformen mit kontrollierter Freisetzung sowie auch an neuen Verfahren zur Herstellung von Darreichungsformen mit kontrollierter Freisetzung.

Es war daher Aufgabe der vorliegenden Erfindung, Trägerpellets und ein Verfahren zu deren Herstellung bereitzustellen, die eine kontrollierte Freisetzung des beladenen, arzneilich wirksamen Bestandteils ermöglichen und die Nachteile der aus dem Stand der Technik bekannten Systeme nicht aufweisen.

Diese Aufgabe wird durch das Verfahren mit den Merkmalen des Anspruchs 1, die Trägerpellets mit den Merkmalen des Anspruchs 8 und die pharmazeutische Formulierung mit den Merkmalen des Anspruchs 9 gelöst. Die weiteren abhängigen Ansprüche zeigen vorteilhafte Weiterbildungen auf. Anspruch 10 gibt eine erfindungsgemäße Verwendung an.

Erfindungsgemäß wird ein Verfahren zur Herstellung von Trägerpellets für einen Wirkstoff bereitgestellt, bei dem

a) eine flüssige Formulierung durch Lösen und/oder Dispergieren von mindestens einem physiologisch verträglichen pH-Regulator in mindestens einem Lösungsmittel oder Emulsionsmittel hergestellt wird,

b) die flüssige Formulierung mittels Düsen in eine Wirbelschicht- oder Strahlschicht-Anlage eingebracht wird,

c) durch Sprühgranulation in der Anlage, bei der mittels eines Trocknungsgasstroms das Lösungsmittel verdampft wird, im wesentlichen sphärische Trägerpellets gebildet werden und

d) die Trägerpellets aus der Anlage kontinuierlich ausgetragen werden.

Es ist bevorzugt, dass ein pH-Regulator eingesetzt wird, der im physiologischen Umfeld dahingehend regulierend wirkt, dass der pH-Wert erniedrigt bzw. erhöht wird und damit die Bioverfügbarkeit von arzneilich wirksamen Bestandteilen ermöglicht oder verstärkt wird. Dies kann aber auch dadurch erreicht werden, dass der pH-Regulator eine stabilisierende Funktion, z.B. beim Einsatz eines Puffersystems als pH-Regulator, aufweist.

Vorzugsweise ist der pH-Regulator eine organische Säure, wobei diese besonders bevorzugt ausgewählt ist aus der Gruppe bestehend aus $C_1$-$C_{18}$-Mono-, Di- und Tricarbonsäuren sowie deren Mischungen. Beispielhafte Vertreter dieser Gruppe sind Zitronensäure, Bernsteinsäure, Apfelsäure, Fumarsäure, Weinsäure, Sorbinsäure, Adipinsäure, deren Salze und Mischungen. Ebenso ist es möglich, dass als pH-Regulator Ascorbinsäure oder deren Salze eingesetzt werden.

Eine weitere bevorzugte Ausführungsform sieht vor, dass der mindestens eine pH-Regulator ein saures oder basisches Salz ist.

Ist der pH-Regulator ein Puffersystem, so besteht dieses bevorzugt aus einem sauren bzw. basischen Salz zusammen mit einer korrespondierenden Lauge oder Säure. Beispiele hierfür sind Zitronensäure/Zitrat oder Weinsäure/Tartrat.

In einer weiteren bevorzugten Variante ist der pH-Regulator eine organische Base, z.B. eine Purinbase oder eine Pyrimidinbase, oder eine Mischung dieser Basen. Die Purinbase ist bevorzugt ausgewählt aus der Gruppe bestehend aus Adenin, Guanin, Hypoxanthin, Xanthin und Mischungen hiervon. Die Pyrimidinbase ist vorzugsweise ausgewählt aus der Gruppe bestehend aus Cytosin, Uracil, Thymin und Mischungen hiervon.

Im Falle, dass der pH-Regulator ein basisches anorganisches Salz ist, ist dieses vorzugsweise ausgewählt aus der Gruppe bestehend aus $NaHCO_3$, $K_2CO_3$, $Na_2CO_3$, $KHCO_3$, $Ca(OH)_2$, CaO, Phosphaten sowie Mischungen hiervon.

Vorzugsweise enthält die Formulierung weiterhin mindestens ein physiologisch verträgliches Bindemittel. Dieses Bindemittel ist dabei vorzugsweise ausgewählt aus der Gruppe bestehend aus Methylcellulosen, Hydroxymethylcellulosen, Hydroxypropylmethylcellulosen, Alginaten, Pektinen, Polyvinylpyrrolidonen, Xanthanen sowie anderen Hydrokolloiden und Mischungen hiervon.

Als Lösungsmittel oder Emulsionsmittel werden bevorzugt Wasser oder organische Lösungsmittel verwendet. Als organische Lösungsmittel sind besonders bevorzugt Ethylalkohol, Isopropanol, n-Propanol oder deren Mischungen.

Das Mengenverhältnis von pH-Regulator zu Bindemittel in der flüssigen Formulierung liegt vorzugsweise im Bereich von 50:50 bis 99:1. Eine bevorzugte flüssige Formulierung weist 30 bis 80 Gew.-% des mindestens einen pH-Regulators, 0,5 bis 5 Gew.-% des mindestens einen Bindemittels und 15 bis 69,5 Gew.-% des mindestens einen Lösungsmittels auf.

Die Sprühgranulation kann sowohl in einer Wirbelschichtanlage als auch in einer Strahlschichtanlage erfolgen. Die Temperatur in diesen Anlagen liegt dabei vorzugsweise im Bereich von 5 bis 100 °C. Der in die Beschichtungsanlage eintretende Trocknungsgasstrom weist am Eintritt in die Anlage vorzugsweise eine Temperatur im Bereich von 5 bis 120 °C auf. Als Trocknungsgas kommen insbesondere konditionierte Luft, Stickstoff oder Inertgase, z.B. Edelgase, in Frage.

Erfolgt die Sprühgranulation in einer Wirbelschichtanlage, wird das Trocknungsgas über einen Siebboden zugeführt. Gleichzeitig wird durch oberhalb des Siebbodens angeordnete Düsen die flüssige Formulierung in die Anlage eingebracht.

Erfolgt die Sprühgranulation in einer Strahlschichtanlage, so wird das Trocknungsgas durch am Boden befindliche Längsspalte zugeführt. Die flüssige Formulierung wird über mindestens eine zwischen den Längsspalten angeordnete Düse eingebracht.

Vorzugsweise erfolgt die Einbringung der flüssigen Formulierung durch die Düse von unten nach oben.

Erfindungsgemäß werden ebenso Trägerpellets bereitgestellt, die mindestens einen physiologisch verträglichen pH-Regulator enthalten. Diese Trägerpellets werden nach dem zuvor beschriebenen Verfahren hergestellt.

Die Trägerpellets weisen vorzugsweise einen Durchmesser im Bereich von 50 μm bis 1,5 mm, insbesondere von 90 μm bis 1,2 mm auf.

Die Trägerpellets sind dabei vorzugsweise im Wesentlichen sphärisch. Die Trägerpellets weisen vorzugsweise eine Sphärizität von 0,8 bis 1,0, insbesondere von 0,9 bis 1,0 auf.

Die Sphärizität berechnet sich dabei nach der folgenden Formel:

$$SPHT = \frac{4\pi A}{U^2}$$

mit A = Fläche und U = Umfang.

Die Sphärizität kann mit Geräten zur Partikelgrößen- und Partikelformanalyse mit dynamischer Bildanalyse durchgeführt werden. Ein hierfür geeignetes Gerät ist beispielsweise der CHAMSIZER von Retsch-Technology.

Weiterhin ist es bevorzugt, dass das Verhältnis von Breite zu Länge der Trägerpellets im Bereich von 0,8 bis 1,0, insbesondere von 0,9 bis 1,0 liegt. Das Verhältnis Breite zu Länge berechnet sich dabei nach der folgenden Formel:

$$b/l = \frac{\min(x_C)}{\max(x_{Fe})}$$

mit $x_{Fe}$ = Feret-Durchmesser und $x_C$ = maximale Breite des Partikels.

Auch das Breite-Länge-Verhältnis lässt sich z.B. mit dem genannten CAMSIZER bestimmen.

Vorzugsweise handelt es sich bei den erfindungsgemäßen Trägerpellets um dichte Trägerpellets, was eine Gewichtsreduzierung gegenüber Extrusionspellets bedeutet.

Die Trägerpellets weisen im Wesentlichen die gleiche Partikelgröße auf, d.h. es liegt ein enger Streubereich hinsichtlich der Partikelgröße vor.

Vorzugsweise enthalten die Trägerpellets mindestens ein physiologisch verträgliches Bindemittel. Dieses Bindemittel ist dabei vorzugsweise ausgewählt aus der Gruppe bestehend aus Methylcellulosen, Hydroxymethylcellulosen, Hydroxypropylmethylcellulosen, Alginaten, Pektinen, Polyvinylpyrrolidonen, Xanthanen sowie anderen Hydrokolloiden sowie Mischungen hiervon.

Erfindungsgemäß wird ebenso eine pharmazeutische Formulierung enthaltend die zuvor beschriebenen Trägerpellets und mindestens einen Wirkstoff bereitgestellt.

Verwendung finden die erfindungsgemäßen Trägerpellets als Trägerstruktur für arzneilich wirksame Bestandteile.

**Beispiel 1**

**Herstellung von Dicarbonsäurepellets anhand von**

**D/L-Apfelsäure**

1.1 Herstellen der Sprühlösung

Die Sprühlösung besteht aus gereinigtem Wasser, Methylcellulose und Apfelsäure. Aus dem gereinigten Wasser und Methylcellulose wird eine 4 %-ige Bindelösung hergestellt. Diese wird auf 70 °C temperiert. Danach erfolgt die Zugabe von Apfelsäure unter ständigem Rühren, bis eine vollständige Lösung vorliegt (Anteil gereinigtes Wasser entspricht Anteil Apfelsäure).

1.2 Teilchenausbildung

Die temperierte Sprühlösung wird in die Strahlschichtapparatur (ProCell) im Bottom Spray-Verfahren eingedüst. Es erfolgt ein stetiger Partikelaufbau durch Verdüsen der Feststofflösung im Hauptluftstrom. Dieser besteht aus zwei Teilströmen, die durch Spaltöffnungen, längs durch den Prozessraum führend, realisiert werden. Der Partikelaufbau geschieht durch das Abdampfen des Lösungsmittels Wasser, Apfelsäure und Methylcellulose verbleiben als Teilchen getrocknet im Luftstrom. Durch das definierte Strömungsprofil des Apparates trennen sich die Partikel im oberen Prozessraum aus dem zentralen Luftstrom und fließen seitlich, bedingt durch Schwerkraft und der Sogwirkung des Hauptluftstromes, zum Prozessgaseintritt zurück. Dort werden sie erneut mit dem Hauptluftstrom mitgerissen und kontinuierlich mit Feststoff aus der Sprühlösung beschichtet. Die Prozessluft ist konditioniert.

Während des kontinuierlichen Einbringens des Feststoffgemisches über Verdüsung, erfolgt gleichzeitig die Entnahme von sauren Pellets. Die Apfelsäurepellets werden für die gewünschte Korngröße fraktioniert.

Dabei kann Unterkorn und aufbereitetes Oberkorn dem Prozess zurückgeführt werden. Endprodukt ist ein homogenes, annähernd sphärisches Apfelsäurepellet mit einer gleichmäßigen Oberflächenstruktur.

Das erfindungsgemäße Verfahren zeichnet sich insbesondere durch die nachfolgend dargestellten Aspekte aus:

1. Verfahren zur Herstellung von Trägerpellets für einen Wirkstoff, bei dem

a) eine flüssige Formulierung durch Lösen und/oder Dispergieren von mindestens einem physiologisch verträglichen pH-Regulator in mindestens einem Lösungsmittel oder Emulsionsmittel hergestellt wird,

b) die flüssige Formulierung mittels Düsen in eine Wirbelschicht- oder Strahlschicht-Anlage eingebracht wird,

c) durch Sprühgranulation in der Anlage, bei der mittels eines Trocknungsgasstroms das Lösungsmittel verdampft wird, im wesentlichen sphärische Trägerpellets gebildet werden und

d) die Trägerpellets aus der Anlage ausgetragen werden.

2. Verfahren nach Aspekt 1, bei dem der mindestens eine pH-Regulator im physiologischen Umfeld dahingehend regulierend wirkt, dass der pH-Wert erniedrigt oder erhöht wird und damit die Bioverfügbarkeit von arzneilich wirksamen Bestandteilen ermöglicht oder verstärkt wird.

3. Verfahren nach einem der Aspekte 1 oder 2, bei dem der pH-Regulator eine organische Säure, insbesondere ausgewählt aus der Gruppe bestehend aus Ascorbinsäure oder $C_1$-$C_{18}$-Mono-, Di- und Tricarbonsäuren sowie Mischungen hiervon, enthält oder hieraus besteht.

4. Verfahren nach Aspekt 3, bei dem die organische Säure ausgewählt ist aus der Gruppe bestehend aus Zitronensäure, Bernsteinsäure, Apfelsäure, Fumarsäure, Weinsäure, Sorbinsäure, Adipinsäure und Mischungen hiervon.

5. Verfahren nach einem der Aspekte 1 bis 4, bei dem der mindestens eine pH-Regulator ein saures oder basisches Salz ist.

6. Verfahren nach einem der Aspekte 1 bis 5, bei dem der pH-Regulator ein Puffersystem aus einem sauren oder basischen Salz zusammen mit einer korrespondierenden Lauge oder Säure enthält oder hieraus besteht und den pH-Wert stabilisiert.

7. Verfahren nach Aspekt 6, bei dem das Puffersystem aus Zitronensäure und einem Zitrat oder aus Weinsäure und einem Tartrat besteht.

8. Verfahren nach einem der Aspekte 1 bis 7, bei dem der pH-Regulator den im physiologischen Umfeld pH-erhöhend, pH-erniedrigend oder pH-stabilisierend wirkt.

9. Verfahren nach einem der Aspekte 1 bis 8, bei dem der pH-Regulator eine organische Base, insbesondere eine Purinbase oder Pyrimidinbase, oder eine Mischung von diesen enthält oder hieraus besteht.

10. Verfahren nach Aspekt 9, bei dem die Purinbase ausgewählt ist aus der Gruppe bestehend aus Adenin, Guanin, Hypoxanthin, Xanthin und Mischungen hiervon.

11. Verfahren nach Aspekt 9, bei dem die Pyrimidinbase ausgewählt ist aus der Gruppe bestehend aus Cytosin, Uracil, Thymin und Mischungen hiervon.

12. Verfahren nach einem der Aspekte 1 bis 11, bei dem der pH-Regulator ein basisches anorganisches Salz, insbesondere $NaHCO_3$, $K_2CO_3$, $Na_2CO_3$, $KHCO_3$, $Ca(OH)_2$, CaO, Phosphate sowie Mischungen hiervon, enthält oder hieraus besteht.

13. Verfahren nach einem der Aspekte 1 bis 12, bei dem die Formulierung mindestens ein physiologisch verträgliches Bindemittel enthält.

14. Verfahren nach Aspekt 13, bei dem das Bindemittel ausgewählt ist aus der Gruppe bestehend aus Methylcellulosen, Hydroxymethylcellulosen, Hydroxypropylmethylcellulosen, Alginaten, Pektinen, Polyvinylpyrrolidonen, Xanthanen sowie anderen Hydrokolloiden und Mischungen hiervon.

15. Verfahren nach einem der Aspekte 1 bis 14, bei dem als Lösungsmittel oder Emulsionsmittel Wasser und/oder ein organisches Lösungsmittel, insbesondere Ethylalkohol, Isopropanol, n-Propanol oder Mischungen von diesen eingesetzt werden.

16. Verfahren nach einem der Aspekte 1 bis 15, bei dem in der flüssigen Formulierung das Mengenverhältnis von pH-Regulator zu Bindemittel im Bereich von 50:50 bis 99:1 liegt.

17. Verfahren nach einem der Aspekte 1 bis 16, bei dem die flüssige Formulierung 30 bis 80 Gew.-% des mindestens einen pH-Regulators, 0,5 bis 5 Gew.-% des mindestens einen Bindemittels und 15 bis 69,5 Gew.-% des mindestens einen Lösungsmittels enthält.

18. Verfahren nach einem der Aspekte 1 bis 17, bei dem die Temperatur in der Anlage im Bereich von 5 bis 100 °C liegt.

19. Verfahren nach einem der Aspekte 1 bis 18, bei dem der Trocknungsgasstrom beim Eintritt in die Anlage eine Temperatur im Bereich von 5 bis 120 °C aufweist.

20. Verfahren nach einem der Aspekte 1 bis 19, bei dem als Trocknungsgas Luft, Stickstoff oder Inertgase eingesetzt wird.

21. Verfahren nach einem der Aspekte 1 bis 20, die Sprühgranulation in einer Wirbelschichtanlage erfolgt, in die über einen Siebboden das Trocknungsgas zugeführt und durch oberhalb des Siebbodens angeordneten Düsen die flüssige Formulierung eingebracht wird.

22. Verfahren nach einem der Aspekte 1 bis 21, bei dem die Sprühgranulation in einer Strahlschichtanlage erfolgt, in die über in der unteren Hälfte der Anlage angeordnete Längsspalte das Trocknungsgas zugeführt und durch mindestens eine zwischen den Längsspalten angeordnete Düse die flüssige Formulierung eingebracht wird.

23. Verfahren nach Aspekt 22, dadurch gekennzeichnet, dass die Einbringung der flüssigen Formulierung durch die Düse von unten nach oben erfolgt.

Die Erfindung betrifft zudem Trägerpellets, die insbesondere durch die nachfolgenden Aspekte gekennzeichnet sind:

24. Trägerpellets enthaltend mindestens einen physiologisch verträglichen pH-Regulator erhältlich nach dem Verfahren nach einem der Aspekte 1 bis 23.

25. Trägerpellets nach Aspekt 24, wobei die Trägerpellets einen Durchmesser im Bereich von 50 µm bis 1,5 mm, insbesondere von 90 µm bis 1,2 mm aufweisen.

26. Trägerpellets nach einem der Aspekte 24 oder 25, wobei die Trägerpellets im Wesentlichen sphärisch sind.

27. Trägerpellets nach einem der Aspekte 24 bis 26, wobei die Trägerpellets eine Sphärizität von 0,8 bis 1,0, insbesondere von 0,9 bis 1,0 aufweisen.

28. Trägerpellets nach einem der Aspekte 24 bis 27, wobei die Trägerpellets ein Breite-Länge-Verhältnis von 0,8 bis 1,0, insbesondere von 0,9 bis 1,0 aufweisen.

29. Trägerpellets nach einem der Aspekte 24 bis 28, wobei die Trägerpellets dichte Trägerpellets sind.

30. Trägerpellets nach einem der Aspekte 24 bis 29, wobei die Trägerpellets im Wesentlichen die gleiche Größe aufweisen.

Außerdem betrifft die vorliegende Erfindung eine pharmazeutische Formulierung, die durch die nachfolgenden Aspekte gekennzeichnet ist:

31. Pharmazeutische Formulierung enthaltend Trägerpellets nach einem der Aspekte 24 bis 30 und mindestens einen Wirkstoff.

Ein weiterer Aspekt der vorliegenden Erfindung betrifft die Verwendung der erfindungsgemäßen Trägerpellets, wie nachfolgend dargestellt:

32. Verwendung von Trägerpellets nach einem der Aspekte 24 bis 30 als Trägerstrukturen für arzneilich wirksame Bestandteile.

## Patentansprüche

1. Verfahren zur Herstellung von Trägerpellets für einen Wirkstoff, bei dem

a) eine flüssige Formulierung durch Lösen und/oder Dispergieren von mindestens einem physiologisch verträglichen pH-Regulator in mindestens einem Lösungsmittel, das aus Wasser und/oder einem organischen Lösungsmittel ausgewählt ist, hergestellt wird,
b) die flüssige Formulierung mittels Düsen in eine Wirbelschicht- oder Strahlschicht-Anlage eingebracht wird,
c) durch Sprühgranulation in der Anlage, bei der mittels eines Trocknungsgasstroms das Lösungsmittel verdampft wird, im wesentlichen sphärische Trägerpellets gebildet werden und
d) die Trägerpellets aus der Anlage ausgetragen werden,
**dadurch gekennzeichnet, dass** der pH-Regulator ausgewählt ist aus der Gruppe bestehend aus

i) $C_1$-$C_{18}$-Mono-, Di- und Tricarbonsäure, sowie Mischungen hiervon;
ii) Puffersystem aus einem sauren oder basischen Salz zusammen mit einer korrespondierenden Lauge oder Säure, wobei das Puffersystem den pH-Wert stabilisiert;
iii) organische Base; und
iv) basisches anorganisches Salz.

2. Verfahren nach einem der Ansprüche 1, **dadurch gekennzeichnet, dass** die $C_1$-$C_{18}$-Mono-, Di- und/oder Tricarbonsäure ausgewählt ist aus der Gruppe bestehend aus Zitronensäure, Bernsteinsäure, Apfelsäure, Fumarsäure, Weinsäure, Sorbinsäure, Adipinsäure und Mischungen hiervon.

3. Verfahren nach einem der Ansprüche 1 oder 2, **dadurch gekennzeichnet, dass** das Puffersystem aus Zitronensäure und einem Zitrat oder aus Weinsäure und einem Tartrat besteht.

4. Verfahren nach einem der Ansprüche 1 bis 3, **dadurch gekennzeichnet, dass** die organische Base eine Purinbase oder Pyrimidinbase, oder eine Mischung von diesen, enthält oder hieraus besteht, wobei die Purinbase ausgewählt ist aus der Gruppe bestehend aus Adenin, Guanin, Hypoxanthin, Xanthin und Mischungen hiervon.

5. Verfahren nach einem der Ansprüche 1 bis 4, **dadurch gekennzeichnet, dass** das basische anorganische Salz $NaHCO_3$, $K_2CO_3$, $Na_2CO_3$, $KHCO_3$, $Ca(OH)_2$, CaO, Phosphate, sowie Mischungen hiervon, enthält oder hieraus besteht.

6. Verfahren nach einem der Ansprüche 1 bis 5, **dadurch gekennzeichnet, dass** als Lösungsmittel oder Emulsionsmittel Ethylalkohol, Isopropanol, n-Propanol oder Mischungen von diesen eingesetzt werden.

7. Verfahren nach einem der Ansprüche 1 bis 6, **dadurch gekennzeichnet, dass** die Sprühgranulation in einer Wirbelschichtanlage erfolgt, in die über einen Siebboden das Trocknungsgas zugeführt und durch oberhalb des Siebbodens angeordneten Düsen die flüssige Formulierung eingebracht wird oder dass die Sprühgranulation in einer Strahlschichtanlage erfolgt, in die über in der unteren Hälfte der Anlage angeordnete Längsspalte das Trocknungsgas zugeführt und durch mindestens eine zwischen den Längsspalten angeordnete Düse die flüssige Formulierung eingebracht wird.

8. Trägerpellets, erhältlich nach dem Verfahren nach einem der Ansprüche 1 bis 7, **dadurch gekennzeichnet, dass** die Trägerpellets mindestens einen physiologisch verträglichen pH-Regulator enthalten.

9. Pharmazeutische Formulierung enthaltend Trägerpellets nach Anspruch 8 und mindestens einen Wirkstoff.

10. Verwendung von Trägerpellets nach Anspruch 8 als Trägerstrukturen für arzneilich wirksame Bestandteile.

**Europäisches Patentamt**
**European Patent Office**
**Office européen des brevets**

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 262 198 A1 (PHARMA PASS S A [FR] PHARMA PASS FRANCE S A S [FR]) 4. Dezember 2002 (2002-12-04) * Beispiel 1 * | 1-5,7-10 | INV. A61K9/16 A61K47/12 |
| X | WO 97/16078 A1 (GIVAUDAN ROURE INT [CH]; MENZI HEINI [CH]; PERREN MATTHIAS [CH]; RINGG) 9. Mai 1997 (1997-05-09) * Seite 8, Zeilen 16-21 * | 8-10 | |
| X | US 5 902 844 A (WILSON EDWARD S [US]) 11. Mai 1999 (1999-05-11) * Beispiele 1-4 * | 1-5,7-10 | |
| X | ONEDA F ET AL: "The effect of formulation variables on the dissolution and physical properties of spray-dried microspheres containing organic salts", POWDER TECHNOLOGY, ELSEVIER SEQUOIA, LAUSANNE, CH, Nr. 130, 1. Januar 2003 (2003-01-01), Seiten 377-384, XP002429927, ISSN: 0032-5910 * das ganze Dokument * | 8-10 | |
| X | US 6 492 024 B1 (WALTER KIM TORBEN [US]) 10. Dezember 2002 (2002-12-10) * Beispiel 8 * | 1-5,7-10 | RECHERCHIERTE SACHGEBIETE (IPC)  A61K |
| X | US 2005/163855 A1 (CHO CHEONG W [KR] ET AL) 28. Juli 2005 (2005-07-28) * Absätze [0032], [0050] - [0062]; Beispiele 8-9 * | 1-10 | |
| X | JP 2001 294524 A (TAISHO PHARMA CO LTD) 23. Oktober 2001 (2001-10-23) * Beispiele 1-2 * | 1-5,7-10 | |

-/--

Der vorliegende Recherchenbericht wurde für alle Patentansprüche erstellt

| Recherchenort | Abschlußdatum der Recherche | Prüfer |
|---|---|---|
| Den Haag | 28. Oktober 2015 | Schwald, Claudia |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...........................................................................................
& : Mitglied der gleichen Patentfamilie, übereinstimmendes
    Dokument

EPO FORM 1503 03.82 (P04C03)

**Europäisches
Patentamt
European
Patent Office
Office européen
des brevets**

## EUROPÄISCHER RECHERCHENBERICHT

Nummer der Anmeldung

EP 15 17 2814

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,P | EP 1 958 520 A2 (SYMRISE GMBH & CO KG [DE]) 20. August 2008 (2008-08-20) * Absätze [0015], [0075], [0077] * ----- | 1-5,7-10 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 28. Oktober 2015 | Schwald, Claudia |

EPO FORM 1503 03.82 (P04C03)

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-10-2015

| Im Recherchenbericht angeführtes Patentdokument | Datum der Veröffentlichung | Mitglied(er) der Patentfamilie | Datum der Veröffentlichung |
|---|---|---|---|
| EP 1262198 A1 | 04-12-2002 | AT 265231 T | 15-05-2004 |
| | | DE 60200416 D1 | 03-06-2004 |
| | | DE 60200416 T2 | 03-03-2005 |
| | | DK 1262198 T3 | 16-08-2004 |
| | | EP 1262198 A1 | 04-12-2002 |
| | | ES 2222436 T3 | 01-02-2005 |
| | | PT 1262198 E | 30-09-2004 |
| | | TR 200401848 T4 | 21-09-2004 |
| | | US 2002192290 A1 | 19-12-2002 |
| WO 9716078 A1 | 09-05-1997 | DE 59609238 D1 | 27-06-2002 |
| | | EP 0859554 A1 | 26-08-1998 |
| | | ES 2175130 T3 | 16-11-2002 |
| | | JP 3657007 B2 | 08-06-2005 |
| | | JP 2000513204 A | 10-10-2000 |
| | | US 6056949 A | 02-05-2000 |
| | | WO 9716078 A1 | 09-05-1997 |
| US 5902844 A | 11-05-1999 | AU 2571499 A | 16-08-1999 |
| | | CA 2319514 A1 | 05-08-1999 |
| | | EP 1058556 A1 | 13-12-2000 |
| | | JP 2002501903 A | 22-01-2002 |
| | | NO 20003832 A | 29-09-2000 |
| | | US 5902844 A | 11-05-1999 |
| | | WO 9938527 A1 | 05-08-1999 |
| US 6492024 B1 | 10-12-2002 | AT 260708 T | 15-03-2004 |
| | | DE 60008636 D1 | 08-04-2004 |
| | | DE 60008636 T2 | 10-03-2005 |
| | | DK 1064990 T3 | 24-05-2004 |
| | | EP 1064990 A1 | 03-01-2001 |
| | | ES 2213560 T3 | 01-09-2004 |
| | | JP 4679696 B2 | 27-04-2011 |
| | | JP 2001070779 A | 21-03-2001 |
| | | US 6492024 B1 | 10-12-2002 |
| US 2005163855 A1 | 28-07-2005 | AU 2005206063 A1 | 04-08-2005 |
| | | BR PI0507094 A | 19-06-2007 |
| | | CA 2554588 A1 | 04-08-2005 |
| | | CN 1921837 A | 28-02-2007 |
| | | EP 1737430 A1 | 03-01-2007 |
| | | JP 2007519714 A | 19-07-2007 |
| | | KR 20050077381 A | 02-08-2005 |
| | | RU 2342926 C2 | 10-01-2009 |
| | | US 2005163855 A1 | 28-07-2005 |
| | | WO 2005070397 A1 | 04-08-2005 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT**
**ÜBER DIE EUROPÄISCHE PATENTANMELDUNG NR.**

EP 15 17 2814

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-10-2015

| Im Recherchenbericht angeführtes Patentdokument | Datum der Veröffentlichung | Mitglied(er) der Patentfamilie | Datum der Veröffentlichung |
|---|---|---|---|
| | | ZA    200607118 A | 30-04-2008 |
| JP 2001294524    A | 23-10-2001 | KEINE | |
| EP 1958520    A2 | 20-08-2008 | EP    1958520 A2<br>US    2008199592 A1 | 20-08-2008<br>21-08-2008 |

EPO FORM P0461

Für nähere Einzelheiten zu diesem Anhang : siehe Amtsblatt des Europäischen Patentamts, Nr.12/82